Europäisches Patentamt

European Patent Office

Office européen des brevets

(11) Veröffentlichungsnummer: **0 421 310 A1**

## (12) EUROPÄISCHE PATENTANMELDUNG

(21) Anmeldenummer: 90118764.1

(22) Anmeldetag: 29.09.90

(51) Int. Cl.5: **A61B 17/22**

(30) Priorität: 02.10.89 DE 3932840

(43) Veröffentlichungstag der Anmeldung:
10.04.91 Patentblatt 91/15

(84) Benannte Vertragsstaaten:
**BE DE FR GB NL**

(71) Anmelder: **Richard Wolf GmbH**
**Pforzheimer Strasse 32**
**W-7134 Knittlingen(DE)**

(72) Erfinder: **Krauss, Werner**

Beethovenstr. 84
W - 7134 Knittlingen(DE)
Erfinder: **Wurster, Helmut**
Mozartstr. 20
W - 7519 Oberderdingen(DE)
Erfinder: **Belikan, Thomas**
Brahmsweg 4
W - 7134 Knittlingen(DE)

(74) Vertreter: **Wilcken, Thomas, Dipl.-Ing. et al**
**Musterbahn 1**
**W-2400 Lübeck(DE)**

(54) **Vorrichtung für die Stosswellen-Therapie.**

(57) Es ist eine Vorrichtung für die Stoßwellen-Therapie mit einem elektroakustischen Therapiewandler (3) zur Erzeugung von im Ultraschall liegenden Stoßwellen beschrieben. Die Stoßwellen sind über eine Flüssigkeits-Vorlaufstrecke (5) mit einer diese abschließenden Membran (7) auf den Körper des Patienten übertragbar, wobei dieser liegend auf einem Behandlungstisch (1) gebettet ist. Zur Vermeidung einer Keimverschleppung und Übertragung von Krankheitserregern ist die auswechselbare Membran (7) mit ihrem Randbereich lösbar am Behandlungstisch (1) befestigt, so daß die mit dem Patientenkörper in Kontakt tretende Membran (7) als Einmalartikel konzipierbar ist und für jeden Patienten eine neue Membran verwendet werden kann.

FIG. 1

EP 0 421 310 A1

# VORRICHTUNG FÜR DIE STOSSWELLEN-THERAPIE

Die Erfindung betrifft eine Vorrichtung für die Stoßwellen-Therapie mit einem elektroakustischen Therapiewandler zur Erzeugung von im Ultraschallbereich liegenden Stoßwellen, die über eine Flüssigkeit-Vorlaufstrecke und eine diese Vorlaufstrecke abschließende Membran auf den Körper des Patienten übertragbar sind, wobei die elastische Membran ein Fenster verschließt, daß in einem den Patienten liegend aufnehmenden Behandlungstisch ausgebildet ist.

Für den im Erfindungsbetreff angegebenen Verwendungszweck sind Einrichtungen verschiedener Konzepte bekannt.

So ist es bekannt, in einer mit Kopplungsflüssigkeit gefüllten Wanne einen Stoßwellengenerator anzuordnen und einen auf einer Liege gebetteten Patienten in diese Wanne so einzutauchen, daß der Fokus des Stoßwellengenerators auf das zu zertrümmernde Konkrement ausgerichtet ist (EP-PS 00 84 093).

Aus der DE-AS 23 51 247 ist eine Einrichtung eines anderen Konzeptes bekannt, bei der der Stoßwellengenerator in einem Brennpunkt einer Fokussierkammer von der Gestalt eines Teils eines Rotationsellipsoides untergebracht ist, welche Kammer durch eine elastische Membran verschlossen ist. Die Fokussierkammer wird mit dieser Membran luftspaltlos am Körper des Patienten angelegt und zwar so, daß sich das Konkrement im zweiten Brennpunkt des Ellipsoides befindet. Die entsprechende Ausrichtung kann nach der EP-A1 131 654 durch axiale Verschiebung der durch eine Membran verschlossenen Fokussierkammer in einem Kopplungszylinder erfolgen, der an den Körper des Patienten angelegt wird und ebenfalls durch eine Membran abgeschlossen ist, wobei sich zwischen den beiden Membranen und in der Fokussierkammer eine Kopplungsflüssigkeit befindet.

Aus der DE-OS 32 20 751 ist es schließlich bekannt, einen Stoßwellengenerator mit einer flexiblen Flüssigkeits-Vorlaufstrecke zu verwenden, die durch eine an den Körper des Patienten anschmiegbare Folie abgeschlossen ist. Die Ankopplung erfolgt manuell unter Vermittlung eines Ankoppelgels.

Es ist die Aufgabe der Erfindung, die Vorrichtung nach dem letztgenannten Stand der Technik dahingehend zu verbessern, daß eine Keimverschleppung und Übertragung von Krankheitserregern vermieden wird. Dabei soll die Ankoppelung an den Körper des Patienten auf einfache Weise und so erfolgen können, daß ein im wesentlichen verlustfreier Übergang der Schallstrahlung aus der Vorlaufstrecke zum Patientenkörper hin erreicht wird.

Diese Aufgabe wird erfindungsgemäß dadurch gelöst, daß die auswechselbare Membran mit ihrem Randbereich lösbar am Behandlungstisch befestigt ist.

Die damit erzielbaren Vorteile bestehen insbesondere darin, daß die mit dem Patientenkörper in Kontakt tretende Membran als Einmalartikel konzipierbar ist, so daß für jeden Patienten eine neue Membran verwendet werden kann.

Für die sichere und leicht lösbare Fixierung der Membran in der Gebrauchslage bieten sich verschiedene Lösungen an. So kann die Membran mit ihrem Randbereich an dem Behandlungstisch pneumatisch festlegbar sein oder der Randbereich der Membran auf einer das Fenster in dem Behandlungstisch umlaufenden, an Unterdruck anschließbaren Mulde plaziert werden. Diese Mulde kann auch auf einem nach oben vorstehenden Polster ausgebildet sein. Als Membran kann ein vorgefertigter Folienabschnitt oder eine mehrere Membranen bevorratende Folienbahn verwendet werden, die abschnittweise über das Fenster bzw. die Vorlaufstrecke bewegbar ist. Bewährt hat sich für die Membran eine Dicke von der Größenordnung von 50 bis 500μm.

Für die Erzielung einer verlustarmen Ankoppelung des Therapiewandlers an den Patientenkörper ist Voraussetzung, daß sich bei dem Membranwechsel in der Vorlaufstrecke sammelndes Gas entfernt wird. Das kann dadurch geschehen, daß eine unterhalb der Membran und innerhalb der Vorlaufstrecke wirkende Vorrichtung zum Absaugen von Gas aus der Vorlaufstrecke vorgesehen wird. Diese Vorrichtung zum Absaugen kann darin bestehen, daß am oberen Randbereich des das Fenster umlaufenden Teiles des Behandlungstisches Ausnehmungen eingearbeitet sind, über die Gas in der Vorlaufstrecke absaugbar ist, oder eine Ringleitung vorgesehen ist, deren nach innen und oben weisende Oberfläche perforiert ausgeführt ist. Zur Durchführung der Absaugung ist bei diesen Ausführungen eine Durchwölbung der Membran nach unten erforderlich, so daß sich dann die Absaugung an der höchstgelegenen Stelle der Membran befindet und die sich dort sammelnden Gasblasen zu erfassen vermag.

Eine andere Vorgehensweise ermöglicht die Verwendung eines aus dem Zentrum des Therapiewandlers ausfahrbaren Teleskoprohres, an dessen frei aufragendem Ende ein perforierter Absaugkörper angeordnet ist. Der Vorteil dieser Ausführung besteht darin, daß durch das Ausfahren gegen die Membran dieser pyramidenartig verformt wird, so daß etwa vorhandene Gasblasen direkt dem an der höchsten Stelle befindlichen Absaugkörper zustreben. Der gleiche Effekt ist erreichbar mit einem radial in die Vorlaufstrecke mündenden, kurbelartig geformten Absaugrohr mit einem an seinem freien Ende angeordneten, perforierten Absaugkörper, wobei das Absaugrohr um den radial geführten Teil schwenkbar ist. Wird die Membran aus einem mikroporösen und gasdurchlässigen, jedoch nicht wasserdurchlässigen Werkstoff, beispielsweise Polyurethan, gefertigt, so kann auf eine gezielte Endgasung der Vorlaufstrecke verzichtet werden.

Die Erfindung wird nachstehend anhand von in den Zeichnungen dargestellten Ausführungsbeispielen näher erläutert. Es zeigen:

Figur 1 eine Anordnung mit einem Vorrat an Membranen in Form einer abschnittweise bewegbaren Folienbahn,

Figur 2 eine Ausführungsform für die lösbare Fixierung der die Vorlaufstrecke abschließenden Membran,

Figur 3 eine Ausführungsvariante für die Fixierung der Membran,

Figur 4 eine Draufsicht auf das Ausführungsbeispiel nach Figur 3,

Figur 5 eine Ausführung für die Entgasung der Vorlaufstrecke,

Figur 6 eine Ausführungsvariante der Vorrichtung für eine Zweckbestimmung entsprechend Figur 5,

Figur 7 eine weitere Ausführungsvariante der Vorrichtung entsprechend Figur 6.

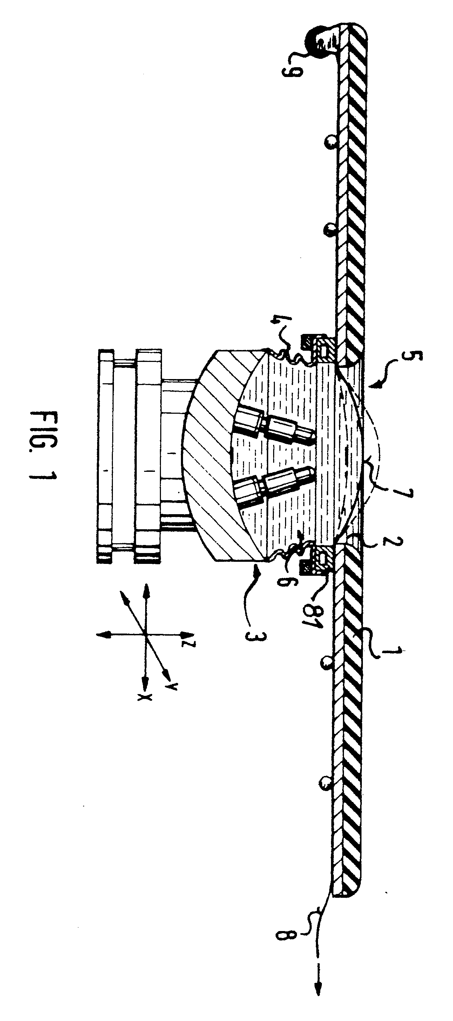

Wie am besten aus Figur 1 ersichtlich ist umfaßt die Behandlungseinrichtung im wesentlichen einen den Patienten aufnehmenden Behandlungstisch 1 mit einem Fenster 2 vorzugsweise kreisförmigen Querschnitts. Unterhalb des Fensters 2 ist in bekannter Weise ein fokussierender Therapiewandler 3 angeordnet, der in drei Koordinatenachsen relativ zu dem Behandlungstisch 1 verstellbar ist. Der Therapiewandler 3 ist mittels eines flexiblen Gehäuses 4 an der Unterseite des Behandlungstisches 1 angeschlossen, wobei das Gehäuse 4 einerseits die Stoßwellenquelle des Therapiewandlers 3 umschließt und andererseits an dem Randbereich des Fensters 2 abgedichtet befestigt ist. Auf diese Weise wird ein Behältnis gebildet, das mit einem Koppelmedium 6, beispielsweise Wasser, gefüllt die Vorlaufstrecke 5 für die Leitung der von dem Therapiewandler 3 erzeugten Stoßwellen auf den Patientenkörper darstellt. Zur Vermeidung des Körperkontaktes mit dem Koppelmedium 6 ist die Vorlaufstrecke 5 im Bereich des Fensters 2 mit einer Membran 7 abgedeckt, die ihrerseits mit dem Gehäuse 4 abgedichtet verbunden ist.

Nach dem Ausführungsbeispiel gemäß Figur 1 wird die Membran 7 aus einer Folienbahn 8 gebildet, die von einer Vorratsrolle 9 längs der Unterseite des Behandlungstisches 1 über das Fenster 2 ziehbar ist. Die Folienbahn 8 ist dabei zwischen der Unterseite des Behandlungstisches 1 und einem gegen diese in geeigneter Weise anpreßbaren Klemmring geführt, der zum Zwecke des Austauschs der Membran 7 gelöst wird, so daß die Folienbahn 8 vorbewegt und anschließend ein unbenutzter Abschnitt eingespannt werden kann. Das für eine einwandfreie Funktion erforderliche Absaugen eventuell in der Vorlaufstrecke 5 befindlicher Gasblasen wird weiter unten beschrieben.

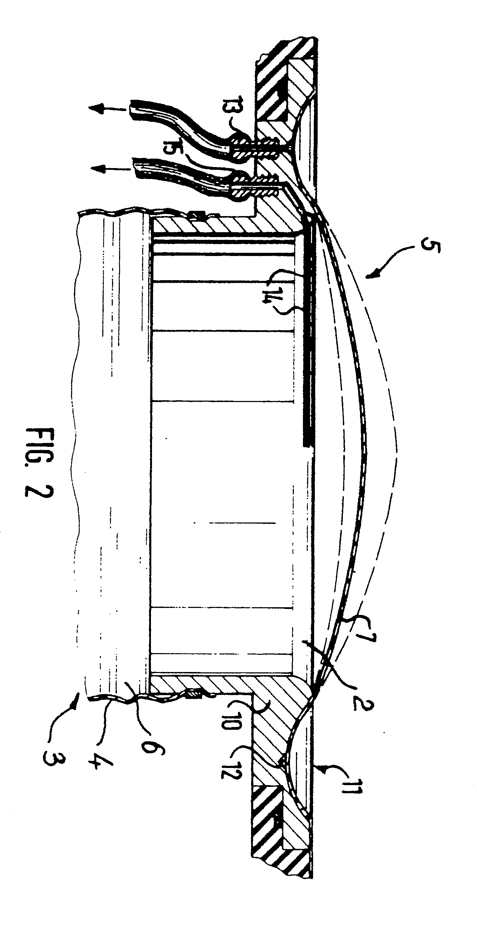

Die Ausführung der die Vorlaufstrecke 5 abschließenden Membran 7 gemäß Figur 2 macht Gebrauch von einem vorgefertigten Folienabschnitt, der zwischen dem oberen Wandlerende und dem Randbereich des Fensters 2 des Behandlungstisches 1 durch ein Ringelement 10 pneumatisch festgelegt wird. Dazu hat der Ringelement 10 eine nutförmige Mulde 11, in deren Grund eine Ringnut 12 angeordnet ist, die über einen Schlauchanschluß 13 mit einer nicht dargestellten Unterdruckquelle in Verbindung steht. Durch Evakuierung der Ringnut 12 wird der Folienabschnitt in die Mulde 11 gesaugt und damit flüssigkeitsdicht festgelegt und ist durch Abstellen der Unterdruckquelle leicht wieder lösbar. Etwa eingefangene Gasblasen können durch im oberen Bereich des Ringelementes 10 eingebrachte schlitzförmige Ausnehmungen 14 über einen mit diesen in Verbindung stehenden Absaugstutzen 15 abgesaugt werden, wenn die Membran 7 nach unten durchgewölbt wird.

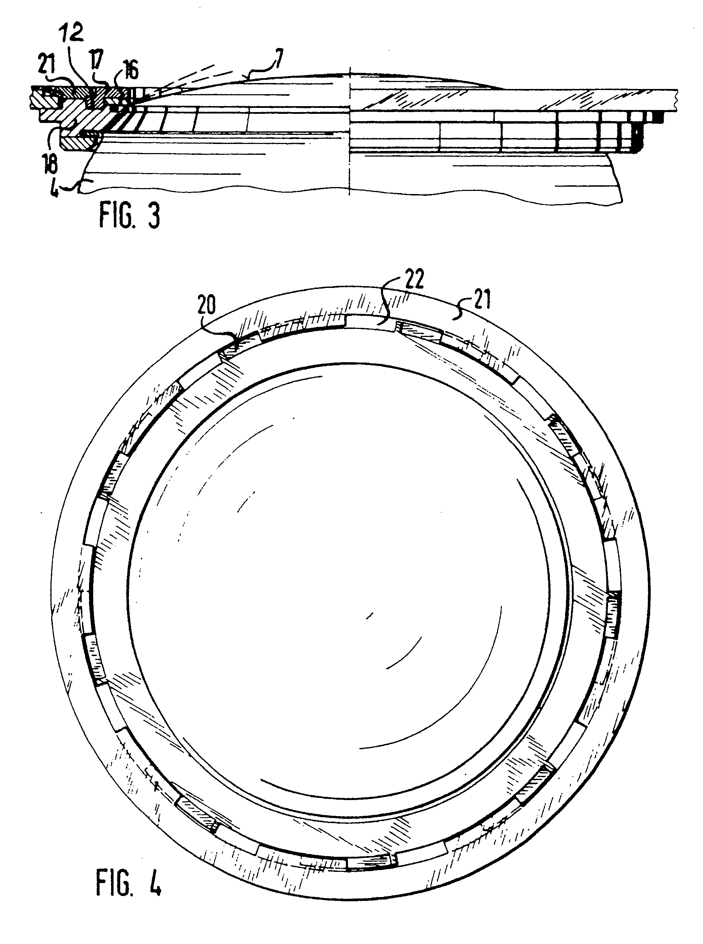

Eine andere Möglichkeit der lösbaren Festlegung einer vorgefertigten Membran 7 zeigt Figur 3. Hier ist die Membran 7 an einem Ring 16 befestigt, der zwischen einem Bajonettring 17 und einem Tragring 18 festgelegt werden kann. Dazu wird die Membran 7 nach Entfernen des Bajonettringes 17 mit ihrem Ring 16 in eine Ausdrehung in dem Tragring 18 eingelegt und der Bajonettring 17 aufgelegt. Dazu wird dieser mit an seinem Umfang befindlichen Stegelemencen 20 in einen die Öffnung des Fensters 2 umgebenden Haltering 21 mit zu den Stegsegmenten 20 komplementären Ausnehmungen 22 eingelegt (Figur 4) und durch Verdrehen axial verspannt.

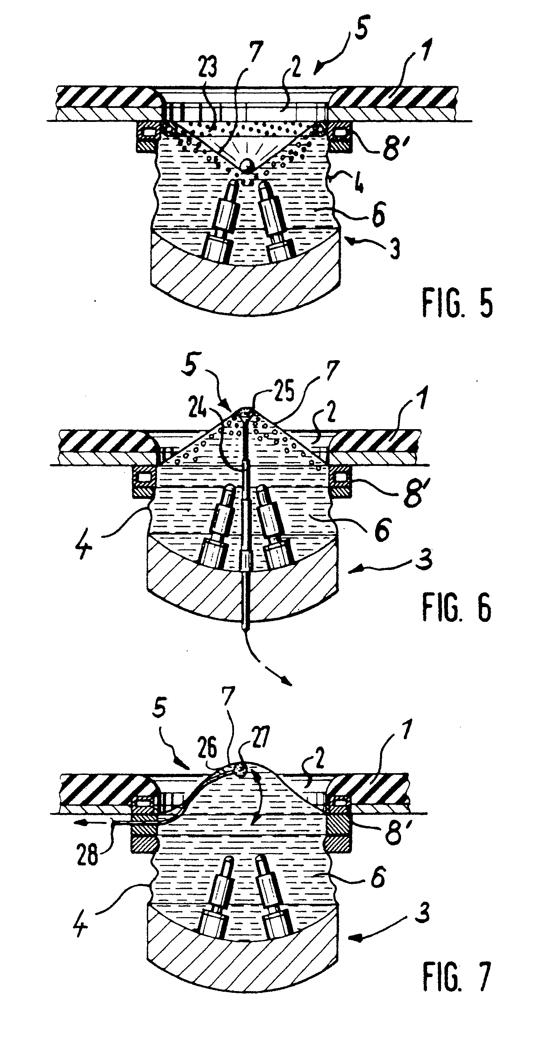

Für die Entgasung des Kopplungsmediums bieten sich verschiedene Vorgehensweisen und Konzepte an. So kann gemäß Figur 5 im oberen Randbereich des Fensters 2 eine dieses umlaufende Ringleitung 23 vorgesehen sein, deren nach innen und oben weisende Oberfläche perforiert ausgeführt ist und die mit einer Unterdruckquelle verbindbar ist. Für die Absaugung der Gasblasen kann die Membran 7 zentral beispielsweise durch ein Gewicht nach unten ausgewölbt werden, so daß die Gasblasen der Ringleitung 23 zustreben, die in dieser Verformung der Membran 7 die höchstgelegene Stelle der Vorlaufstrecke 5 bildet.

Gemäß der in Figur 6 dargestellten Ausführung ist ein aus dem Zentrum der Stoßwellenquelle des Therapiewandlers 3 auf geeignete Weise ausfahrbares Teleskoprohr 24 vorgesehen, an dessen frei aufragendem Ende ein perforierter Absaugkörper 25 angeordnet ist. Durch Ausfahren des Teleskoprohres 24 tritt der Absaugkörper 25 mit der Membran 7 in Kontakt und wölbt diese nach oben aus, so daß sich die Gasblasen im Bereich des Absaugkörpers 25 sammeln und dort abgesaugt werden können.

Eine weitere Ausführung einer Entgasungseinrichtung ist in Figur 7 gezeigt. Sie umfaßt ein radial in die Vorlaufstrecke 5 mündendes, kurbelartig ausgeformtes Absaugrohr 26, das mit einem an seinem freien Ende angeordneten, perforierten Absaugkörper 27 versehen ist. Das Absaugrohr 26 ist um den radial geführten Teil 28 um 90° schwenkbar, so daß der Absaugkörper 27 zwischen einer seitlichen Stellung unmittelbar unterhalb der entspannten Membran 7 in eine Stellung bringbar ist, die eine Durchwölbung der Membran 7 entsprechend Figur 6 ermöglicht.

Für eine Vorfixierung der die Membran 7 bildenden vorgefertigten Folienabschnitte vor der endgültigen Festlegung durch die vorbeschriebenen möglichen Handhabungen, können die Folienabschnitte in ihrem Randbereich mit einer selbsthaftenden Schicht versehen sein.

## Ansprüche

1. Vorrichtung für die Stoßwellen-Therapie mit einem elektroakustischen Therapiewandler zur Erzeugung von im Ultraschallbereich liegenden Stoßwellen, die über eine Flüssigkeits-Vorlaufstrecke und eine diese Vorlaufstrecke abschließende Membran auf den Körper des Patienten übertragbar sind, wobei die elastische Membran ein Fenster verschließt, das in einem den Patienten liegend aufnehmenden Behandlungstisch ausgebildet ist, dadurch gekennzeichnet, daß die auswechselbare Membran (7) mit ihrem Randbereich lösbar am Behandlungstisch (1) befestigt ist.

2. Vorrichtung nach Anspruch 1, dadurch gekennzeichnet, daß der Randbereich der Membran (7) pneumatisch am Behandlungstisch (1) festlegbar ist.

3. Vorrichtung nach Anspruch 1 oder 2, dadurch gekennzeichnet, daß der Randbereich der Membran (7) auf einer das Fenster (2) umlaufenden, an Unterdruck anschließbaren Mulde (11) des Behandlungstisches (1) liegt.

4. Vorrichtung nach Anspruch 3, dadurch gekennzeichnet, daß die Mulde (11) in einem das Fenster (2) umlaufenden, nach oben vorstehenden Polster ausgebildet ist.

5. Vorrichtung nach Anspruch 1, dadurch gekennzeichnet, daß der verstärkt ausgebildete Rand der Membran (7) auf einem Tragring (18) liegt und zwischen dem Tragring und einem Bajonettring (17) eingespannt ist, der über eine Bajonettverbindung lösbar mit dem Tragring verbunden ist.

6. Vorrichtung nach einem der Ansprüche 1 bis 5, dadurch gekennzeichnet, daß die Membran (7) ein vorgefertigter Folienabschnitt ist.

7. Vorrichtung nach einem der Ansprüche 1 bis 5, dadurch gekennzeichnet, daß mehrere Membranen (7) als Folienbahn (8) bevorratet sind, die abschnittweise über das Fenster (2) bzw. die Vorlaufstrecke (5) bewegbar ist.

8. Vorrichtung nach einem der Ansprüche 1 bis 6, dadurch gekennzeichnet, daß die Membran (7) eine Dicke in der Größenordnung von etwa 50 bis 500 $\mu$m hat.

9. Vorrichtung nach einem der Ansprüche 1 bis 8, dadurch gekennzeichnet, daß eine unterhalb der Membran (7) und innerhalb der Vorlaufstrecke (5) wirkende Vorrichtung zum Absaugen von Gas aus der Vorlaufstrecke (5) vorgesehen ist.

10. Vorrichtung nach Anspruch 9, dadurch gekennzeichnet, daß im oberen Randbereich des das Fenster (2) umlaufenden Teiles des Behandlungstisches (1) Ausnehmungen (14) eingearbeitet sind, über die Gas in der Vorlaufstrecke (5) absaugbar ist.

11. Vorrichtung nach Anspruch 9, dadurch gekennzeichnet, daß die Vorrichtung zum Absaugen von Gas aus der Vorlaufstrecke (5) aus einer das Fenster (2) am oberen Randbereich umlaufenden Ringleitung (23) besteht, deren nach innen und oben weisende Oberfläche perforiert ausgeführt ist.

12. Vorrichtung nach Anspruch 9, dadurch gekennzeichnet, daß ein aus dem Zentrum des Therapiewandlers (3) ausfahrbares Teleskop (24) vorgesehen ist, an dessen frei aufragendem Ende ein perforierter Absaugkörper (25) angeordnet ist.

13. Vorrichtung nach Anspruch 9, dadurch gekennzeichnet, daß ein radial in die Vorlaufstrecke (5) mündendes, kurbelartig geformtes Absaugrohr (26) mit einem an seinem freien Ende angeordneten perforierten Absaugkörper (27) vorgesehen ist, und daß das Absaugrohr (26) um den radial geführten Teil (28) schwenkbar ist.

14. Vorrichtung nach Anspruch 9, dadurch gekennzeichnet, daß die Vorrichtung zum Absaugen von Gas aus der Vorlaufstrecke (5) darin besteht, daß die Membran (7) aus einem mikroporösen und gasdurchlässigen, jedoch nicht wasserdurchlässigen Werkstoff, beispielsweise Polyurethan gefertigt ist.

FIG. 1

FIG. 2

FIG. 3

FIG. 4

FIG. 5

FIG. 6

FIG. 7

**Europäisches Patentamt**

# EUROPÄISCHER RECHERCHENBERICHT

Nummer der Anmeldung

**EP 90 11 8764**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | WO-A-8 703 797 (EISENMENGER) <br> * Seite 4, Zeile 15 - Seite 5, Zeile 2; Anspruch 8; Figur 1 * <br> – – – | 1,2,7,9 | A 61 B <br> 17/22 |
| Y | FR-A-2 192 797 (BOUSQUET) <br> * Anspruch 1; Figur 1 * <br> – – – | 1,2,7,9 | |
| A | EP-A-0 234 366 (SIEMENS) <br> * Spalte 7, Zeilen 41-52; Figur 8 * <br> – – – | 1,5 | |
| A | EP-A-0 226 041 (DORNIER) <br> * Seite 3, Zeilen 6-17; Figur 4 * <br> – – – – – | 1 | |

| | RECHERCHIERTE SACHGEBIETE (Int. Cl.5) |
|---|---|
| | A 61 B |

Der vorliegende Recherchenbericht wurde für alle Patentansprüche erstellt

| Recherchenort | Abschlußdatum der Recherche | Prüfer |
|---|---|---|
| Den Haag | 08 Januar 91 | MOERS R.J. |